# EUROPEAN PATENT APPLICATION

(11) **EP 3 841 973 A1**
(43) Date of publication of application: **30.06.2021**
(21) Application number: 19219218.5
(22) Date of filing: 23.12.2019
(51) Int. Cl.: A61B 5/055

(54) **MAGNETIC RESONANCE BREAST SUPPORT**

(71) Applicant: Koninklijke Philips N.V., 5656 AG Eindhoven (NL)
(72) Inventor: BECK, Gabriele Marianne, 5656 AE Eindhoven (NL); POSSANZINI, Cecilia, 5656 AE Eindhoven (NL)
(74) Representative: Philips Intellectual Property & Standards

(57) **Abstract**

Disclosed herein is a medical instrument (100, 200, 300, 400, 500, 600, 900) comprising a subject support (102) configured for supporting a subject (110) in a Fowler's position during a magnetic resonance imaging examination. The subject support comprises a leg support region (104) configured for supporting a leg region of the subject horizontally. The subject support further comprises a thoracic support (106) configured for supporting an upper body region of the subject. The subject support is configured such that the thoracic support is inclined (108) with respect to the leg support region to hold the subject in the Fowler's position. The medical instrument further comprises a breast support (114). The breast support comprises a planar support surface (116) configured for supporting breasts of the subject. The breast support is connected to the subject support. The support surface is configured for being horizontal during the magnetic resonance imaging examination.

## Description

### FIELD OF THE INVENTION

The invention relates to Magnetic Resonance Imaging, in particular to the imaging of the breast region.

### BACKGROUND OF THE INVENTION

A large static magnetic field is used by Magnetic Resonance Imaging (MRI) scanners to align the nuclear spins of atoms as part of the procedure for producing images within the body of a patient. This large static magnetic field is referred to as the B0 field or the main magnetic field. Various quantities or properties of the subject can be measured spatially using MRI. MRI is able to distinguish between various types of soft tissue. Anatomical regions comprised of soft tissue, such as the breasts, may therefore be studied using MRI.

United States patent application US 2013/0218000 A1 discloses a novel MRI coil configuration for breast cancer imaging in preparation for radiation therapy planning and treatment. Both an adjustable anterior and posterior coil sets are described. The coil sets are supported and configured so as not to affect the accurate and repeatable positioning of the patient and breasts. The coil sets are removable so that they can be extracted before radiation therapy treatment commences.

### SUMMARY OF THE INVENTION

The invention provides for a medical instrument and a method in the independent claims. Embodiments are given in the dependent claims.

Breast cancer has commonly been screened for using mammography. Because of its ability to image and differentiate different types of soft tissue, magnetic resonance imaging (MRI) would be an ideal imaging modality to screen for breast cancer. A difficulty has been that common MRI subject supports for breast cancer have the subject in a prone position where the breasts are hanging. The subject therefore much climb into the subject support. Elderly and sick patients have trouble doing this. Subjects also lie on their ribs which may be uncomfortable. Additionally, some subjects may not physically fit into the magnetic resonance imaging coil.

Embodiments of the invention may provide for an improved means of imaging the breasts with a magnetic resonance imaging system. Embodiments provide for a subject support configured for supporting a subject in a Fowler's position or semi-sitting position. A breast support with a planar support surface may be positioned under the breasts to support them. This may have the advantage of being able to reduce breast motion during an MRI examination. This may also be useful for magnetic resonance guidance of radiotherapy and biopsy. Additionally, the subject is then held in a position where the lymph nodes and the breasts can be conveniently imaged in a single volume. Putting the subject in the Fowler's position reduces the size of the single volume or region of interest for imaging both the lymph nodes and the breasts.

The subject support may be used in a variety of ways. In some embodiment, coil elements may be included directly within the breast support to image the breasts and/or the axillary lymph nodes. In other embodiments a flexible imaging coil may be placed over the breast and/or axillary lymph node region. This flexible imaging coil could for example be a surface coil that is placed over the subject, a wearable coil that the subject puts on before the examination, or even a support which secures the breasts to the planar support surface.

It should be noted that references to breasts herein are also refer to apparatus and imaging of a single breast. A subject who has, for example, undergone a mastectomy of a single breast may use the apparatus and methods described herein.

In one aspect the invention provides for a medical instrument that comprises a subject support configured for supporting a subject in a Fowler's position during a magnetic resonance imaging examination. The subject support comprises a leg support region configured for supporting a leg region of the subject horizontally during the magnetic resonance imaging examination. The subject support further comprises a thoracic support configured for supporting the upper body region of the subject. The upper body region is superior to the leg region. The subject support is configured such that the thoracic support is inclined with respect to the leg support region to hold the subject in the Fowler's position during the magnetic resonance imaging examination. The medical instrument further comprises a breast support.

The breast support comprises a planar support surface configured for supporting the breasts of the subject. The breast support is connected to the subject support. The support surface is configured for being horizontal during the magnetic resonance imaging examination. This embodiment may be beneficial because the breast support can hold the breasts in a defined position where they are supported against the gravitational force. This may aid in imaging the breasts without compressing them.

The mechanical attachment of the breast support may be performed in several different ways. There may be mechanical linkages which connect the breast support to the thoracic support and/or the leg support. For example, the breast support may be constructed so that the distance between the planar support surface and the leg region support is adjustable. This may be for example accomplished in different ways. Linkages may be used. The breast support may also be attached to the thoracic support using a slider or fasteners that may be positioned in different locations.

In another embodiment the breast support is rigidly attached to the subject support.

In another embodiment the thoracic support is inclined with respect to the leg support region between 10° and 40°. This embodiment may be beneficial because it may enable the breast support to support the breasts gravitationally.

In another embodiment the thoracic support is inclined with respect to the leg support between 15° and 30°. This embodiment may be beneficial because it may provide for an improved means of fitting the subject and support within the bore of a magnetic resonance imaging system.

In another embodiment the breast support comprises two edge supports each connected to opposite edges of the thoracic support. The planar support surface is supported by and is in between the two edge supports. The planar support surface is operable for sliding horizontally between the two edge supports to adjust support of the breasts. This embodiment may be beneficial because it may provide for an effective means of adjusting the breast support.

In another embodiment the two edge supports are arm supports also. This may be beneficial because then both the arms and the breasts are supported. This for example may aid in imaging the breasts and the lymph nodes simultaneously.

In another embodiment the medical instrument further comprises a breast restraint configured for fixing the breasts on the breast support. This embodiment may be beneficial because it may prevent the breasts from moving during the magnetic resonance examination.

In another embodiment the breast restraint is a planar breast restraint configured for compressing the breasts against the breast support. This embodiment may be beneficial because it may be useful for example simulating a mammogram.

In some embodiments the subject support is configured to hold the breasts in the same position as a magnetic resonance imaging examination, for a biopsy, and or a mammogram. This may facilitate comparing the different imaging modalities.

In another embodiment the breast restraint is a three-dimensional printed breast restraint configured for fixing the location of the breasts against the breast support. This embodiment may be beneficial because the breast restraint can be modeled to match to the breasts of the subject and can restrain them in a non-compressive way. This may also for example be useful in fixing the location of the breasts for radiotherapy. This may aid in obtaining the same position every time during multiple radiotherapy sessions.

In another embodiment the breast restraint comprises a flexible receptacle comprising breast cups configured to restrain the breasts. This embodiment may be beneficial because it may provide for an effective means of restraining the breasts with a great deal of comfort for the subject. The flexible receptacle may for example be implements using a flexible material such as plastic or a fabric.

In another embodiment the breast restraint further comprises a multi-channel magnetic resonance imaging coil configured for imaging the breasts and axillary lymph nodes of the subject during the magnetic resonance imaging examination. This may be beneficial because it may provide for an effective means of detecting if the subject has cancer or other disease of the breast which is also in the lymph nodes.

In another embodiment the medical instrument further comprises a surface coil configured for being placed adjacent or over the breasts and/or the auxiliary lymph nodes.

In another embodiment the multi-channel magnetic resonance imaging coil may be a magnetic resonance microscopy coil.

In another embodiment the medical instrument further comprises a multi-channel magnetic resonance imaging coil embedded within the breast support. The multi-channel magnetic resonance imaging coil is configured for imaging the breasts and the axillary lymph nodes of the subject during the magnetic resonance imaging examination. This may be beneficial because the antenna is provided by the breast support and has a consistent geometry. In some examples the supports used to connect the breast support to the subject support may also have antennas in them. This may be for example an arm rest which the subject can rest her arms upon and this may aid in imaging the axillary lymph nodes of the subject.

In another embodiment the medical instrument further comprises a magnetic resonance imaging system configured to acquire magnetic resonance imaging data from an imaging zone during the magnetic resonance imaging examination. The subject support is configured to support the breasts of the subject within the imaging zone.

The subject support may also be adapted to support the axillary lymph nodes of the subject within the imaging zone.

In another embodiment the medical instrument further comprises a memory storing machine-executable instructions and pulse sequence commands. The medical instrument further comprises a processor configured for controlling the medical instrument. Execution of the machine-executable instructions causes the processor to control the magnetic resonance imaging system to position the breasts of the subject within the imaging zone. Execution of the machine-executable instructions further causes the processor to acquire the magnetic resonance imaging data by controlling the magnetic resonance imaging system with the pulse sequence commands. Execution of the machine-executable instructions further causes the processor to reconstruct at least one magnetic resonance image from the magnetic resonance imaging data. This embodiment may be beneficial because the use of the subject support and the breast support may provide for improved quality of magnetic resonance images of the breast region.

In another embodiment the medical instrument further comprises a breast biopsy system. The magnetic resonance imaging system is configured for guiding the breast biopsy system. This embodiment may be beneficial because the breast support may provide for a means of non-compressively supporting the breasts during a magnetic resonance imaging exam. This may make it easier to perform the breast biopsy.

In another embodiment the medical instrument further comprises a radiotherapy system configured for irradiating a target zone. The target zone is within the imaging zone. The memory further contains radiotherapy system control commands configured for controlling targeting of the radiotherapy system. Execution of the machine-executable instructions further causes the processor to adjust the radiotherapy system control commands using the at least one magnetic resonance image. Execution of the machine-executable instructions further causes the processor to irradiate the target zone by controlling the radiotherapy system with the adjusted radiotherapy system control commands. This embodiment may be beneficial because this may provide for better guidance of radiotherapy of the breasts.

In another embodiment the medical instrument comprises a subject support configured for supporting a subject in a Fowler's position during the magnetic resonance imaging examination. The subject support comprises a leg region support configured for supporting the leg region of the subject horizontally when in an operating position. The subject support further comprises a thoracic support configured for supporting an upper body region of the subject. The upper body region is superior to the leg region. The thoracic support is inclined with respect to the leg support region to hold the subject in the Fowler's position.

The medical instrument further comprises a breast support. The breast support comprises a planar support surface configured for supporting the breasts of the subject. The breast support is connected to the subject support. The support surface is horizontal when in the operating position. The medical instrument further comprises a magnetic resonance imaging system configured to acquire magnetic resonance imaging data from an imaging zone. The subject support is configured to support the breasts of the subject within the imaging zone.

The method comprises placing the subject on the subject support. The method further comprises adjusting the breast support such that the planar support surface is under the breasts of the subject and above the heart of the subject. The method further comprises controlling the subject support to move the breasts into the imaging zone. The method further comprises acquiring magnetic resonance imaging data by controlling the magnetic resonance imaging system with pulse sequence commands. The method further comprises reconstructing at least one magnetic resonance image from the magnetic resonance imaging data.

In another embodiment the breast support comprises two edge supports each connected to opposite edges of the thoracic support. The planar support surface is supported by and is in between the two edge supports. The planar support surface is operable for sliding horizontally between the two edge supports to adjust support of the breasts. The method further comprises adjusting the breast support by sliding the planar support horizontally. This embodiment may be beneficial because it provides a very effective means of adjusting the breast support for a subject.

In another embodiment the medical instrument further comprises a fabric breast restraint comprising breast cups and configured to restrain the breasts to the breast support. The fabric breast restraint further comprises a multi-channel magnetic resonance imaging coil configured for imaging the breasts and axillary lymph nodes of the subject. The method further comprises attaching the fabric breast restraint to the breast support to mobilize the breasts and then optionally placing a cushion between the breasts and the axillary lymph nodes. The cushion is formed from a magnetic field homogenizing material

It is understood that one or more of the aforementioned embodiments of the invention may be combined as long as the combined embodiments are not mutually exclusive.

As will be appreciated by one skilled in the art, aspects of the present invention may be embodied as an apparatus, method or computer program product. Accordingly, aspects of the present invention may take the form of an entirely hardware embodiment, an entirely software embodiment (including firmware, resident software, microcode, etc.) or an embodiment combining software and hardware aspects that may all generally be referred to herein as a "circuit," "module" or "system." Furthermore, aspects of the present invention may take the form of a computer program product embodied in one or more computer readable medium(s) having computer executable code embodied thereon.

Any combination of one or more computer readable medium(s) may be utilized. The computer readable medium may be a computer readable signal medium or a computer readable storage medium. A 'computer-readable storage medium' as used herein encompasses any tangible storage medium which may store instructions which are executable by a processor of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the processor of the computing device. Examples of computer-readable storage media include, but are not limited to: a floppy disk, a magnetic hard disk drive, a solid state hard disk, flash memory, a USB thumb drive, Random Access Memory (RAM), Read Only Memory (ROM), an optical disk, a magneto-optical disk, and the register file of the processor. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example, a data may be retrieved over a modem, over the internet, or over a local area network. Computer executable code embodied on a computer readable medium may be transmitted using any appropriate medium, including but not limited to wireless, wire line, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

'Computer memory' or 'memory' is an example of a computer-readable storage medium. Computer memory is any memory which is directly accessible to a processor. 'Computer storage' or 'storage' is a further example of a computer-readable storage medium. Computer storage is any non-volatile computer-readable storage medium. In some embodiments computer storage may also be computer memory or vice versa.

A 'processor' as used herein encompasses an electronic component which is able to execute a program or machine executable instruction or computer executable code. References to the computing device comprising "a processor" should be interpreted as possibly containing more than one processor or processing core. The processor may for instance be a multi-core processor. A processor may also refer to a collection of processors within a single computer system or distributed amongst multiple computer systems. The term computing device should also be interpreted to possibly refer to a collection or network of computing devices each comprising a processor or processors. The computer executable code may be executed by multiple processors that may be within the same computing device or which may even be distributed across multiple computing devices.

Computer executable code may comprise machine executable instructions or a program which causes a processor to perform an aspect of the present invention. Computer executable code for carrying out operations for aspects of the present invention may be written in any combination of one or more programming languages, including an object oriented programming language such as Java, Smalltalk, C++ or the like and conventional procedural programming languages, such as the "C" programming language or similar programming languages and compiled into machine executable instructions. In some instances, the computer executable code may be in the form of a high level language or in a pre-compiled form and be used in conjunction with an interpreter which generates the machine executable instructions on the fly.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present invention are described with reference to flowchart illustrations and/or block diagrams of methods, apparatus (systems) and computer program products according to embodiments of the invention. It is understood that each block or a portion of the blocks of the flowchart, illustrations, and/or block diagrams, can be implemented by computer program instructions in form of computer executable code when applicable. It is further under stood that, when not mutually exclusive, combinations of blocks in different flowcharts, illustrations, and/or block diagrams may be combined. These computer program instructions may be provided to a processor of a general purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks.

These computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks. A 'user interface' as used herein is an interface which allows a user or operator to interact with a computer or computer system. A 'user interface' may also be referred to as a 'human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, pedals, wired glove, remote control, and accelerometer are all examples of user interface components which enable the receiving of information or data from an operator.

A 'hardware interface' as used herein encompasses an interface which enables the processor of a computer system to interact with and/or control an external computing device and/or apparatus. A hardware interface may allow a processor to send control signals or instructions to an external computing device and/or apparatus. A hardware interface may also enable a processor to exchange data with an external computing device and/or apparatus. Examples of a hardware interface include, but are not limited to: a universal serial bus, IEEE 1394 port, parallel port, IEEE 1284 port, serial port, RS-232 port, IEEE-488 port, Bluetooth connection, Wireless local area network connection, TCP/IP connection, Ethernet connection, control voltage interface, MIDI interface, analog input interface, and digital input interface.

A 'display' or 'display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen, Cathode ray tube (CRT), Storage tube, Bi-stable display, Electronic paper, Vector display, Flat panel display, Vacuum fluorescent display (VF), Light-emitting diode (LED) displays, Electroluminescent display (ELD), Plasma display panels (PDP), Liquid crystal display (LCD), Organic light-emitting diode displays (OLED), a projector, and Head-mounted display.

Magnetic Resonance (MR) data is defined herein as being the recorded measurements of radio frequency signals emitted by atomic spins using the antenna of a Magnetic resonance apparatus during a magnetic resonance imaging scan. Magnetic resonance data is an example of medical image data. A Magnetic Resonance Imaging (MRI) image or MR image is defined herein as being the reconstructed two- or three-dimensional visualization of anatomic data contained within the magnetic resonance imaging data. This visualization can be performed using a computer.

### BRIEF DESCRIPTION OF THE DRAWINGS

In the following preferred embodiments of the invention will be described, by way of example only, and with reference to the drawings in which:
- Fig. 1: illustrates an example of a medical instrument;
- Fig. 2: illustrates a further example of a medical instrument;
- Fig. 3: illustrates a further example of a medical instrument;
- Fig. 4: illustrates a further example of a medical instrument;
- Fig. 5: illustrates a further example of a medical instrument;
- Fig. 6: illustrates a further example of a medical instrument;
- Fig. 7: illustrates an example of a breast restraint;
- Fig. 8: illustrates a further example of a medical instrument;
- Fig. 9: illustrates a further example of a medical instrument; and
- Fig. 10: illustrates a method of operating a medical instrument.

### DETAILED DESCRIPTION OF THE EMBODIMENTS

Like numbered elements in these figures are either equivalent elements or perform the same function. Elements which have been discussed previously will not necessarily be discussed in later figures if the function is equivalent.

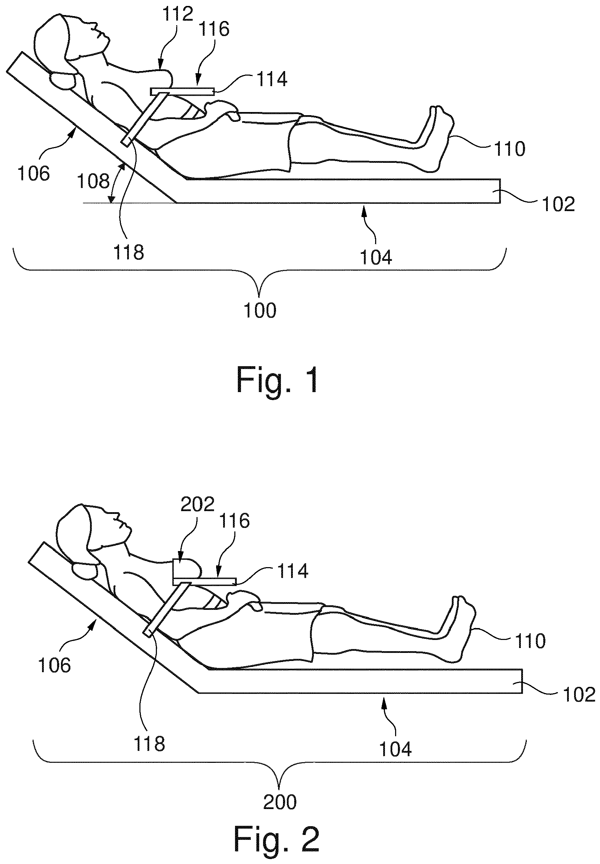

Fig. 1 illustrates an example of a medical instrument 100. The medical instrument 100 comprises a subject support 102 with a leg support region 104 and a thoracic support region 106. The thoracic support region 106 is inclined by an incline angle 108 with respect to the leg support region 104. This holds a subject 110 in a Fowler's position.

There is a breast support 114 that has a planar support surface 116 configured for receiving the breasts 112 of the subject 110. These are able to hold the breasts 112 such that there is minimal compression of the breasts during a magnetic resonance examination.

Fig. 2 shows a further example of medical instrument 200. The medical instrument 200 is similar except that it additionally comprises a breast restraint 202. The breast restraint 202 is configured for fixing the breasts 112 on the planar support surface 116. For example, there may be attachment points or other means of attaching the breast restraint 202. The breast restraint 202 may take different forms in different examples. In some examples the breast restraint may be a planar breast restraint. This may result in the compression of the breasts but it may for example be useful in taking synthetic X-rays that are equivalent to mammograms. In other examples the breast restraint could be a three-dimensional printed breast restraint. This may for example be particularly useful for radiotherapy operations for positioning the breasts in identical positions through multiple radiotherapy sessions. In another example the breast restraint could be a fabric receptacle that comprises breast cups that are configured to restrain the breasts with a minimal compression. These may also include various magnetic resonance antennas that are incorporated into the breast cups and other portions of the fabric receptacles. For example, the fabric of the breast restraint could also contain additional coils such that they can be positioned near the axillary lymph nodes.

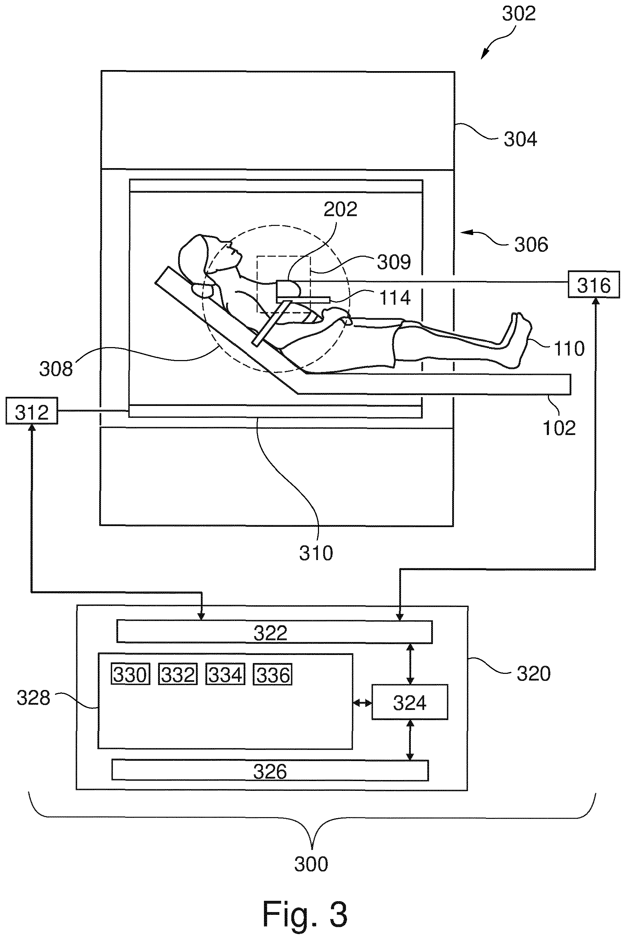

Fig. 3 shows a further example of a medical instrument 300. The medical instrument is similar to the medical instrument depicted in Fig. 2 except it additionally comprises a magnetic resonance imaging system 302. The breast restraint 202 also functions as a multi-element or coil radio frequency coil.

The magnetic resonance imaging system 302 comprises a magnet 304. The magnet 304 is a superconducting cylindrical type magnet with a bore 306 through it. The use of different types of magnets is also possible; for instance it is also possible to use both a split cylindrical magnet and a so called open magnet. A split cylindrical magnet is similar to a standard cylindrical magnet, except that the cryostat has been split into two sections to allow access to the iso-plane of the magnet, such magnets may for instance be used in conjunction with charged particle beam therapy. An open magnet has two magnet sections, one above the other with a space in-between that is large enough to receive a subject: the arrangement of the two sections area similar to that of a Helmholtz coil. Open magnets are popular, because the subject is less confined. Inside the cryostat of the cylindrical magnet there is a collection of superconducting coils.

Within the bore 306 of the cylindrical magnet 304 there is an imaging zone 308 where the magnetic field is strong and uniform enough to perform magnetic resonance imaging. A region of interest 309 is shown within the imaging zone 308. The magnetic resonance data that is acquired typically acquried for the region of interest. The subject support 102 supports a subject 110 within the imaging zone 308 and the region of interest 309.

Within the bore 306 of the magnet there is also a set of magnetic field gradient coils 310 which is used for acquisition of preliminary magnetic resonance data to spatially encode magnetic spins within the imaging zone 308 of the magnet 304. The magnetic field gradient coils 310 connected to a magnetic field gradient coil power supply 312. The magnetic field gradient coils 310 are intended to be representative. Typically magnetic field gradient coils 310 contain three separate sets of coils for spatially encoding in three orthogonal spatial directions. A magnetic field gradient power supply supplies current to the magnetic field gradient coils. The current supplied to the magnetic field gradient coils 310 is controlled as a function of time and may be ramped or pulsed.

Adjacent to the imaging zone 308 is the radio-frequency coil within the breast restraint 202. The radio frequency coi 202 is configured for manipulating the orientations of magnetic spins within the imaging zone 308 and for receiving radio transmissions from spins also within the imaging zone 308. The radio frequency antenna 202 may contain multiple coil elements. The radio frequency antenna may also be referred to as a channel or antenna. The radio-frequency coil 202 is connected to a radio frequency transceiver 316. The radio frequency transceiver 316 may be replaced by a separate transmitter and receiver. It is understood that the radio-frequency coil 202 and the radio frequency transceiver 316 are representative. The radio-frequency coil 202 is intended to also represent a dedicated transmit antenna and a dedicated receive antenna. Likewise the transceiver 316 may also represent a separate transmitter and receivers. The radio-frequency coil 202 may also have multiple receive/transmit elements and the radio frequency transceiver 316 may have multiple receive/transmit channels. For example if a parallel imaging technique such as SENSE is performed, the radio-frequency could 202 will have multiple coil elements.

The magnetic resonance imaging system 302 is further shown as comprising a computer 302 which has a hardware interface 322 that enables the processor 324 to send and receive commands to control the magnetic resonance imaging system 302. The processor 324 is connected to the hardware interface 322, the optional user interface 326 and a memory 328. The hardware interface 322 is any interface which may enable the processor 324 to exchange commands and controls and data with other components of the magnetic resonance imaging system 302. The breast restraint 202, which also functions as a radio frequency coil, is shown as being connected to the transceiver 316. The transceiver 316 and the gradient controller 312 are shown as being connected to the hardware interface 106 of a computer system 102.

The memory 328 may be any memory which is accessible to the processor 324. It may also be an example of a non-transitory storage medium.

The memory 328 is shown as containing machine-executable instructions. The machine-executable instructions 330 contain commands which enable the processor 324 to control the operating and function of the medical instrument 300. The machine-executable instructions 330 may also enable the processor 324 to perform various data processing and image processing tasks. The memory 328 is further shown as containing pulse sequence commands 332. The pulse sequence commands are commands or data which may be converted into such commands that enable the processor 324 to control the magnetic resonance imaging system 302 to acquire magnetic resonance imaging data. The memory 328 is further shown as containing magnetic resonance imaging data 334 that has been acquired by controlling the magnetic resonance imaging system 302 with the pulse sequence commands 332. The memory 328 is further shown as containing a magnetic resonance image 336 that has been reconstructed from the magnetic resonance imaging data 334.

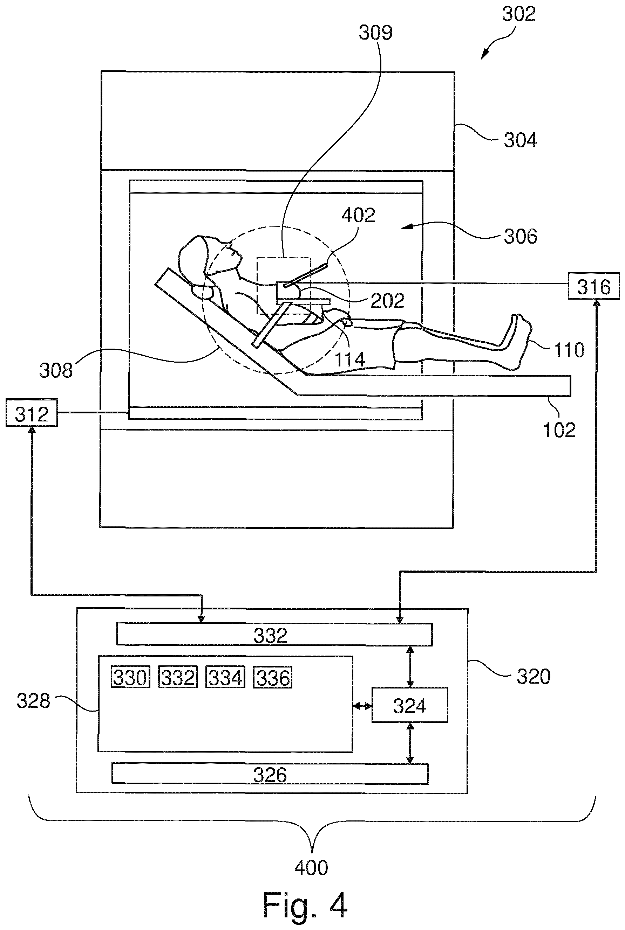

Fig. 4 shows a further example of a medical instrument 400. The medical instrument 400 is similar to the medical instrument 300 depicted in Fig. 3 except that it additionally comprises a breast biopsy system 402. The breast biopsy system may be a manual system or an automated system which may be used for performing a breast biopsy. In particular the biopsy needle or system 402 may be adapted such that its position can be ascertained or determined using the magnetic resonance image 336. By repeatedly acquiring the magnetic resonance image 336 it may enable a healthcare provider to guide the biopsy of the breasts.

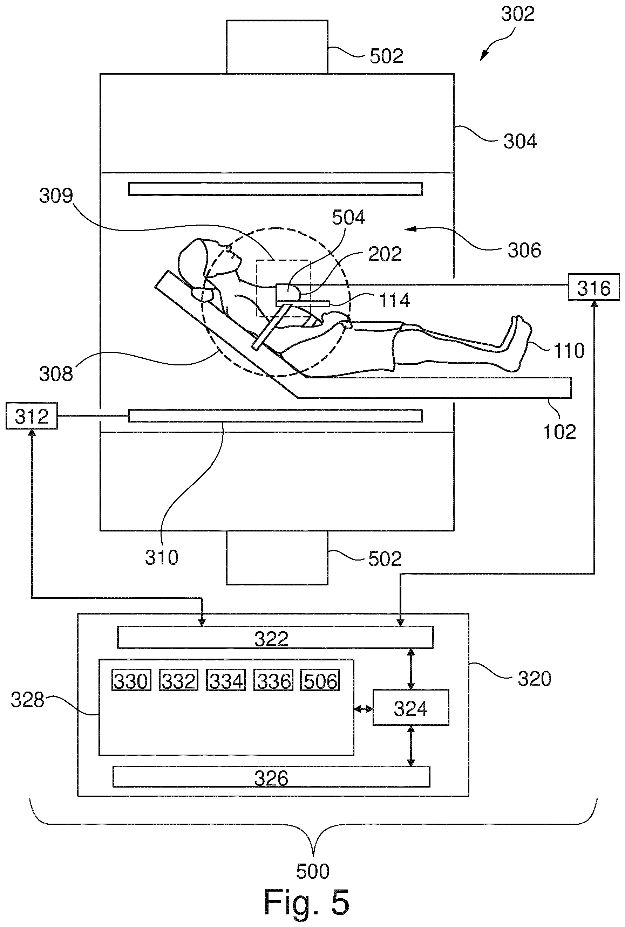

Fig. 5 shows a further example of a medical instrument 500. The medical instrument 500 is similar to the medical instrument 300 depicted in Fig. 3 except that it additionally comprises a radiotherapy system 502. The radiotherapy system 502 could for example be a linear accelerator or other radiotherapy system. The radiotherapy system 502 is configured for irradiating a target zone 504 within the imaging zone 308. The memory 328 is shown as comprising radiotherapy system control commands 506. These commands are adapted for steering the location of the target zone 504. Execution of the machine-executable instructions 330 may cause the processor 324 to adjust the radiotherapy system control commands 506 using the magnetic resonance image 336. By repeatedly acquiring the magnetic resonance image 336 the radiotherapy of the subject 110 may be guided by the magnetic resonance imaging system 302. In some examples, the magnetic resonance imaging coils adjacent to the breast region may be removable to facilitate irradiating the target zone 504.

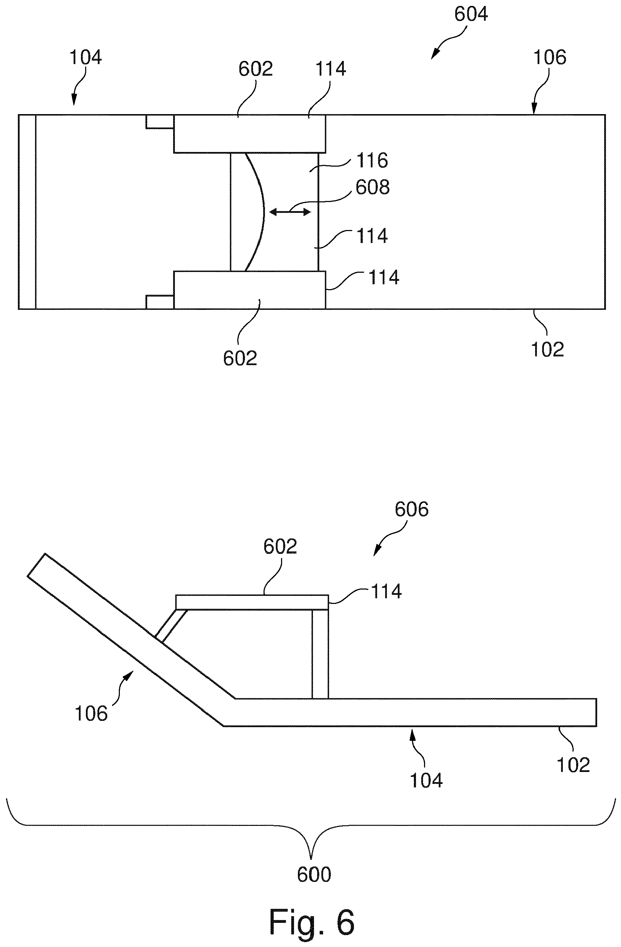

Fig. 6 illustrates a further example of a medical instrument 600. In this example the breast support 114 comprises two edge supports 602 which are each attached to the thoracic support region 106. Fig. 6 shows a top 604 and a side view 606. The planar support surface 116 slides along the edge supports 602 to make it adjustable. The subject can repose in the subject support 102 and then the planar support surface 116 can be comfortably adjusted to fit the particular subject by adjusting the position of the planar support surface 116 along the direction of the arrow 608.

In this example there are mechanical attachments 118 extending to the subject support to rigidly attach the breast support 114 to the subject support.

The edge supports 602 may be arm rests. This may be beneficial, because supporting the arms on the arm rests 602 may facilitate imaging the axillary lymph nodes.

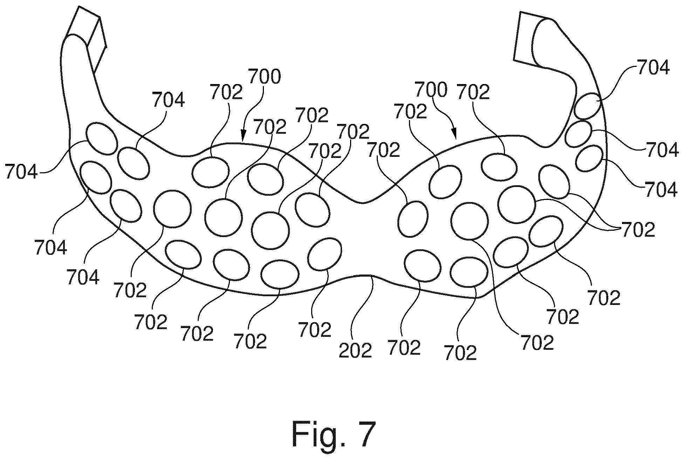

Fig. 7 shows a further example of a breast restraint 202. This breast restraint 202 is made of a flexible material such as a fabric or sheet of plastic and comprises two breast cups 700. Within the breast cups there are breast coil elements 702. There are also additional lymph node coil elements 704 that can be positioned near the axillary lymph nodes of the subject. In some examples, the breast restraint 202 may also comprise connections to the table or thoracic support so that the lymph node coil elements 704 are held in position near the lymph nodes. On each side is a connector 706 which may be used to connect the breast restraint 202 to the breast support.

In other examples, the breast restraint 202 may be wearable. This may have the advantage that the subject can conveniently wear with breast restraint with the coils in advance.

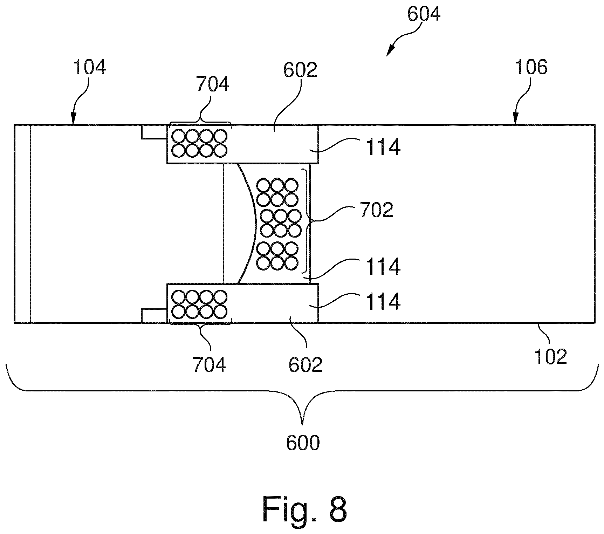

Fig. 8 shows a further example of the breast support 114 from Fig. 6. In this example the planar support surface 116 has been modified by embedding coil elements 702 and 704 within it. In this case the breast support 114 functions as the magnetic resonance imaging coil also. The placement of the lymph node coil elements 704 on the arm rests 602 may facilitate imaging the axillary lymph nodes.

The examples described herein may implement the various coil elements 702, 704 as digital coil elements. For example, the subject support may have a connection that receives a fiber optic connection from the coils 702 or 704. This may have the advantage of improving the SNR of the receive coils 702, 704.

Breast cancer is the most prevalent cancer of women. The golden standard in breast screening and scanning typically is done with different modalities such as ultra-high-resolution Mammography, ultra sound echo and biopsy. MR Breast imaging and screening is very promising but lacks behind in terms of SNR, resolution, B0 and B1 inhomogeneity in the regions of the breast and lymph nodes. Examples may provide for a comfortable and motion robust patient and multi-channel "microscopy" coil setup that enables thin slice, ultra-high-resolution imaging of the breast and lymph nodes avoiding the cardiac region and guaranteeing B0 and B1 homogeneity.

MR Breast imaging and screening of cancer is very promising. It however lags behind with respect to SNR, resolution and motion robustness of the breast and lymph nodes within acceptable scan time. B0 and B1 homogeneity effects can also lead to distortions and saturation effects.

The invention proposes a motion robust patient and multi-channel "microscopy capable" coil setup that enables thin slice, ultra-high resolution imaging of the breast and lymph nodes removing the effect of motion avoiding the cardiac region and providing patient comfort.
Embodiments may contain one or more of the following features:
- Patient and flexible multi-channel "microscopy" coil setup covering breasts and lymph nodes simultaneously with the breasts fixed via a subject support construction to avoid motion and enable digital connection.
- Mechanical fixation of the breast involves a two or more plane fixation, one with a push up effect located under the breast fixed to the subject support and others with a blocking function against breast movement and tissue relaxation.
- Patient positioned in a comfortable half sitting supine position which allows a small scan volume selection covering breast and lymph nodes simultaneously avoiding the heart region
- Use of B0 and B1 field homogenizing material between breast and lymph node

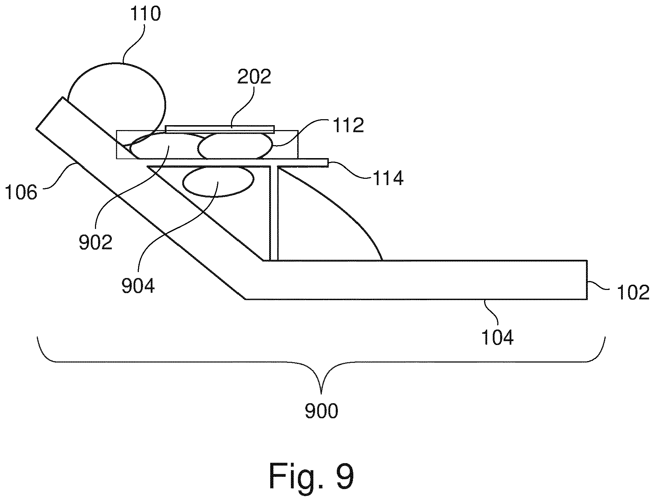

Embodiments may provide for a means to position the patient in a comfortable angled supine position supported by a patient cushion construction fixed to the subject support. The Breasts are positioned on a stable table construction fixed to the patient cushion (see fig. 9 below) or subject support to reduce breathing motion. Extra measure to block breast movements and relaxation effects can be accomplished by a top construction or bra form that flattens the breasts similar to mammography however not compressing them (a breast restraint 202). Secondary, the breasts and lymph nodes are covered by a flexible multi-channel "microscopy" coil set up optimizing the SNR in the breast and lymph node region. The flexible coil setup guarantees a better patient comfort and good coverage into the lymph nodes. The angled patient position enables breast and lymph node scanning within the same ultra-thin imaging plane avoiding the cardiac region. High SNR, ultra-high resolution is guaranteed. Advantage over existing patient and coil set-up where breasts are hanging in a prone patient position is that breasts are fixed and tissue relaxation over time are avoided. SNR and slice coverage may be optimized minimizing the effects from cardiac motion, B0 and B1 effects using homogenizing material. The subject support construction integrates an easy digital connection of the multi-channel coil elements to the subject support.

Embodiments may provide for a multi-channel "microscopy" and patient setup allows the unique capability to provide ultra-high resolution and SNR of the breasts and lymph nodes avoiding the cardiac region with a comfortable half sitting position.

Fig. 9 shows a conceptual diagram of a medical instrument 900. It comprises again a subject support 102 as depicted in the previous Figs. The breast restraint 202 in this case is shown as being a compressive plate for compressing the breasts. The breast support 114 is shown as being positioned below the breasts 112 but above the heart 904. The breast support 114 also is below the lymph nodes 902 of the subject 110. In some examples the subject support 102 may also have connections which provide a digital connection to connect to receive coils such as may be included in the breast support 114 and/or the breast restraint 202.

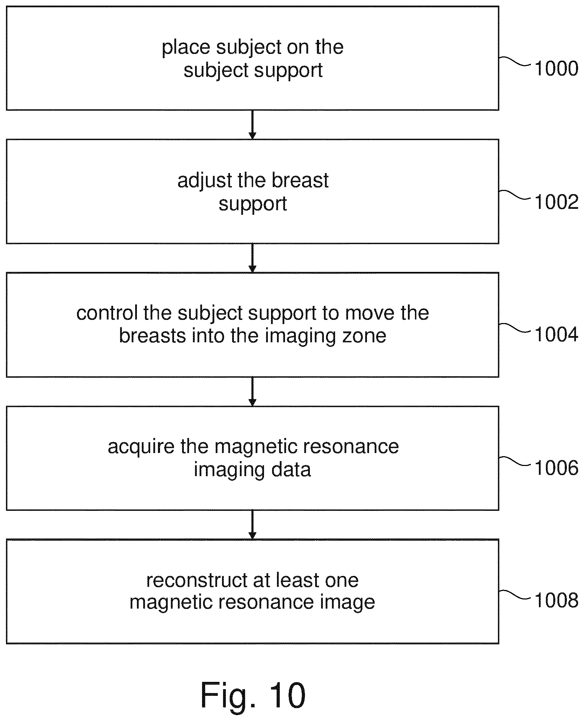

Fig. 10 illustrates a method of operating the medical instrument of Figs. 3, 4, or 5. In step 1000, the subject is placed on the subject support 102. Next in step 1002, the beast support 114 is adjusted such that the planar support surface 116 is under the breasts or breast 112 of the subject 110 and above the heart 904 of the subject. Then in step 1004 the subject support 102 is moved such that the breasts 112 and/or the axillary lymph nodes are within the imaging zone 308. In step 1006 magnetic resonance imaging data 334 is acquired by controlling the magnetic resonance imaging system 302 with pulse sequence commands 332. Finally, in step 1008 at least one magnetic resonance image is reconstructed 1008 from the magnetic resonance imaging data 334.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measured cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

### LIST OF REFERENCE NUMERALS

100 medical instrument
102 magnetic resoance support
104 leg support region
106 thoratic support region
108 incline angle
110 subject
112 breasts
114 breast support
116 planar support surface
118 mechanical attachment
200 medical instrument
202 breast restraint
300 medical instrumnet
302 magnetic resonance imaging system
304 magnet
306 bore of magnet
308 imaging zone
309 region of interest
310 magnetic field gradient coils
312 magnetic field gradient coil power supply
316 transceiver
320 computer
322 hardware interface
324 processor
326 user interface
328 memory
330 machine executable instructions
332 pulse sequence commands
334 magnetic resonance imaging data
336 magnetic resoancne image
400 medical instrument
402 breast biopsy system
500 medical instrument
502 radiotherapy system
504 target zone
506 radiotherapy system control commands
600 medical instrument
602 edge support
604 top view
606 side view
608 direction of motion
700 breast cups
702 breast coil elements
704 lymph node coil elements
706 connection to table
800 coil elements
900 medical instrument
902 lymph nodes
904 heart
1000 placing the subject on the subject support
1002 adjusting the breast support such that the planar support surface is under the breasts of the subject and above the heart of the subject
1004 controlling the subject support to move the breasts into the imaging zone
1006 acquire magnetic resonance imaging data by controlling the magnetic resonance imaging system with pulse sequence commands
1008 reconstruct at least one magnetic resonance image from the magnetic resonance imaging data

## Claims

1. A medical instrument (100, 200, 300, 400, 500, 600, 900) comprising a subject support (102) configured for supporting a subject (110) in a Fowler's position during a magnetic resonance imaging examination, wherein the subject support comprises a leg support region (104) configured for supporting a leg region of the subject horizontally during the magnetic resonance imaging examination, wherein the subject support further comprises a thoracic support (106) configured for supporting an upper body region of the subject, wherein subject support is configured such that the thoracic support is inclined (108) with respect to the leg support region to hold the subject in the Fowler's position during the magnetic resonance imaging examination, wherein the medical instrument further comprises a breast support (114), wherein the breast support comprises a planar support surface (116) configured for supporting breasts of the subject, wherein the breast support is connected to the subject support, wherein the support surface is configured for being horizontal during the magnetic resonance imaging examination.

2. The medical instrument of claim 1, wherein the thoracic support is inclined with respect to the leg support region between 10 degrees and 40 degrees, preferably between 15 degrees and 30 degrees.

3. The medical instrument of claim 2, wherein the breast support comprises two edge supports (602) each connected to opposite edges of the thoracic support, wherein the tow edge supports are preferably arm rests, wherein the planar support surface is supported by and is in between the two edge supports, wherein the planar support surface is operable for sliding horizontally (608) between the two edge supports to adjust support of the breasts.

4. The medical instrument of claim 1, 2, or 3, wherein the medical instrument further comprises a breast restraint (202) configured for fixing the breasts on the breast support.

5. The medical instrument of claim 4, wherein the breast restraint is a planar breast restraint configured for compressing the breasts against the breast support.

6. The medical instrument of claim 4, wherein the breast restraint is a three-dimensional printed breast restraint configured for fixing a location of the breasts against the breast support.

7. The medical instrument of claim 4, wherein the breast restraint comprises a flexible receptable comprising breast cups (700) configured to restrain the breasts.

8. The medical instrument of claim 7, wherein the breast restraint further comprises a multi-channel magnetic resonance imaging coil (702, 704) configured for imaging the breasts (702) and axillary lymph nodes (704) of the subject during the magnetic resonance imaging examination.

9. The medical instrument of any one of claims 1 through 7, wherein the medical instrument comprises a multi-channel magnetic resonance imaging coil (702, 704) imbedded within the breast support, wherein the multi-channel magnetic resonance imaging coil is configured for imaging the breasts (702) and the axillary lymph nodes (704) of the subject during the magnetic resonance imaging examination.

10. The medical instrument of any one of the preceding claims, wherein the medical instrument further comprises a magnetic resonance imaging system (302) configured to acquire magnetic resonance imaging data (334) from an imaging zone (308) during the magnetic resonance imaging examination, and wherein the subject support is configured to support the breasts of the subject within the imaging zone.

11. The medical instrument of claim 10, wherein the medical instrument further comprises:
- a memory (328) storing machine executable instructions (330) and pulse sequence commands (332), wherein execution
- a processor configured for controlling the medical instrument, wherein execution of the machine executable instructions causes the processor to:
- acquire the magnetic resonance imaging data by controlling the magnetic resonance imaging system with the pulse sequence commands; and
- reconstruct at least one magnetic resonance image (336) from the magnetic resonance imaging data.

12. The medical instrument of claim 10 or 11, wherein the medical instrument further comprises a breast biopsy system (402), and wherein the magnetic resonance imaging system is configured for guiding the breast biopsy system.

13. The medical instrument of claim 10 or 11, wherein the medical instrument further comprises a radiotherapy system (502) configured for irradiating a target zone (504), wherein the target zone is within the imaging zone, wherein the memory further contains radiotherapy system control commands (506) configured for controlling targeting of the radiotherapy system, wherein execution of the machine executable instructions further causes the processor to:
- adjust the radiotherapy system control commands using the at least one magnetic resonance image; and
- irradiate the target zone by controlling the radiotherapy system with the adjusted radiotherapy system control commands.

14. A method of operating a medical instrument (100, 200, 300, 400, 500, 600, 900), wherein the medical instrument comprises a subject support (102) configured for supporting a subject (110) in a Fowler's position during a magnetic resonance imaging examination, wherein the subject support comprises a leg region support region 104 configured for supporting a leg region of the subject during the magnetic resonance imaging examination, wherein the subject support further comprises a thoracic support region 106 configured for supporting an upper body region of the subject, wherein the thoracic support is inclined (108) with respect to the leg support region to hold the subject in the partially reclining position;
- a breast support (114), wherein the breast support comprises a planar support surface (116) configured for supporting breasts of the subject, wherein the breast support is connected to the subject support, wherein the support surface is horizontal during the magnetic resonance imaging examination; and
- a magnetic resonance imaging system (302) configured to acquire magnetic resonance imaging data (334) from an imaging zone (308), and wherein the subject support is configured to support the breasts of the subject within the imaging zone;
wherein the method comprises:
- placing (1000) the subject on the subject support;
- adjusting (1002) the breast support such that the planar support surface is under the breasts of the subject and above the heart of the subject;
- controlling (1004) the subject support to move the breasts into the imaging zone;
- acquire (1006) magnetic resonance imaging data (334) by controlling the magnetic resonance imaging system (302) with pulse sequence commands; and
- reconstruct (1008) at least one magnetic resonance image from the magnetic resonance imaging data.

15. The method of claim 14, wherein the medical instrument further comprises a flexible breast restraint comprising breast cups (700) configured to restrain the breasts to the breast support, wherein the flexible breast restraint further comprises a multi-channel magnetic resonance imaging coil (702, 704) configured for imaging the breasts (202) and axillary lymph nodes (902) of the subject, and wherein the method further comprises:
- attaching the flexible breast restraint to the breast support to immobilize the breasts; and
- optionally placing a cushion between the breasts and the axillary lymph nodes, wherein the cushion is formed from a magnetic field homogenizing material.
